# EUROPEAN PATENT SPECIFICATION

(11) **EP 3 228 207 B1**
(45) Date of publication and mention of the grant of the patent: **06.03.2019**
(21) Application number: 15876428.2
(22) Date of filing: 05.01.2015
(51) Int. Cl.: A44C 5/00, G06F 3/01, A61B 5/00, A61B 5/0205, A61B 5/021, A61B 5/024, A61B 5/026, A61B 5/145, A61B 5/01

(54) **DETECTION METHOD FOR WEARABLE DEVICE, AND WEARABLE DEVICE**
DETEKTIONSVERFAHREN FÜR EINE TRAGBARE VORRICHTUNG SOWIE TRAGBARE VORRICHTUNG
PROCÉDÉ DE DÉTECTION POUR DISPOSITIF PORTABLE ET DISPOSITIF PORTABLE

(43) Date of publication of application: 11.10.2017
(73) Proprietor: Huawei Technologies Co. Ltd., Shenzhen, Guangdong 518129 (CN)
(72) Inventor: LI, Liang, Shenzhen Guangdong 518129 (CN)
(74) Representative: Goddar, Heinz J.
(86) International application number: PCT/CN2015/070088
(87) International publication number: WO 2016/109917

(56) References cited:
- CN-A- 101 410 900
- CN-A- 103 536 279
- CN-A- 104 155 897
- CN-U- 203 759 644
- US-A1- 2008 019 218
- US-A1- 2008 045 806
- US-A1- 2012 184 878
- US-A1- 2014 225 730
- US-A1- 2014 366 123

## Description

### TECHNICAL FIELD

The present invention relates to the communications field, and in particular, to a detection method for a wearable device and a wearable device.

### BACKGROUND

A wearable device is a portable device that can be directly worn on a body or integrated into clothes or an accessory of a user. The wearable device is a hardware device that implements multiple functions by means of software support, data exchange, and cloud interaction. In the prior art, detection is mainly performed by using a capacitive sensor. However, during use of the capacitive sensor, when a metal object approaches the capacitive sensor, the capacitive sensor fails and detection precision is relatively low. If whether the wearable device is worn on the user cannot be determined precisely, detection fails or a detection result is not accurate when another object approaches, making it difficult to use the wearable device normally, and causing much inconvenience to the user.

US 2014/366123 A1 discloses a wearable computing device configured for determining whether the wearable computing device is worn by a user based on a single sensor and/or attribute or based on a combination of sensors and/or attributes. For example, the device may determine that the wearable computing device is worn by a user if a threshold skin conductance amount is detected by a sensor or if a motion sensor has detected movement within the past 20 minutes.

US 2008/045806 A1 a method and an apparatus for transmitting physiological and biometric data of a living being, wherein the transmission can be based on the results of a sensing of biometric data, a sensing of histological data and on the determination of a security condition. The apparatus can may comprise a variety of sensor components which record physiological data of the user via optical or acoustic signals.

US 2008/019218 A1 proposes a pulsometer which contains an electronic optical pulse measuring device, an electronic circuit, and a tightening wristband. The electronic optical measuring device includes at least two light sources and at least two receivers, wherein the light sources and the receivers are arranged in the form of a matrix.

Embodiments of the present invention provide a detection method for a wearable device and a wearable device. According to the method, whether a user wears a wearable device can be precisely determined by detecting both a value of a distance between the wearable device and the user and a body characteristic value of the user.

According to a first aspect, an embodiment of the present invention provides a detection method for a wearable device, including:
detecting, by a distance sensor comprised in the wearable device, a value of a distance between the wearable device and a user;
detecting, by a human body characteristic sensor comprised in the wearable device, a body characteristic value of the user;
determining, by a processor comprised in the wearable device, whether the value of the distance between the wearable device and the user is less than or equal to a preset distance threshold; and determining, by the processor, whether the body characteristic value of the user exceeds a preset threshold range of the body characteristic value of the user; and
determining, by the processor, that the wearable device is worn on the user, if the value of the distance between the wearable device and the user is less than or equal to the preset distance threshold and the body characteristic value of the user does not exceed the preset threshold of the body characteristic value of the user.

With reference to the first aspect, in a first possible implementation manner of the first aspect, the method further includes:
determining, by the processor, that the wearable device is not worn on the user, if the value of the distance between the wearable device and the user is greater than the preset distance threshold.

With reference to the first aspect, in a second possible implementation manner of the first aspect, the method further includes:
determining, by the processor, that the wearable device is not worn on the user, if the body characteristic value of the user exceeds the preset threshold of the body characteristic value of the user.

With reference to any one of the first aspect to the second possible implementation manner of the first aspect, in a third possible implementation manner of the first aspect,
the body characteristic value of the user includes one or more of a body temperature value, a heart rate value, a blood glucose value, a blood pressure value, or a blood flow velocity value.

According to a second aspect, an embodiment of the present invention further provides a wearable device, including: a distance sensor, a human body characteristic sensor, and a processor, where
the distance sensor is configured to detect a value of a distance between the wearable device and a user;
the human body characteristic sensor is configured to detect a body characteristic value of the user; and
the processor is configured to: determine whether the value of the distance between the wearable device and the user is less than or equal to a preset distance threshold; determine whether the body characteristic value of the user exceeds a preset threshold range of the body characteristic value of the user; and determine that the wearable device is worn on the user, if the value of the distance between the wearable device and the user is less than or equal to the preset distance threshold and the body characteristic value of the user does not exceed the preset threshold of the body characteristic value of the user.

With reference to the second aspect, in a first possible implementation manner of the second aspect, the processor is further configured to:
determine that the wearable device is not worn on the user, if the value of the distance between the wearable device and the user is greater than the preset distance threshold.

With reference to the second aspect, in a second possible implementation manner of the second aspect, the method further includes:
determining that the wearable device is not worn on the user, if the body characteristic value of the user exceeds the preset threshold of the body characteristic value of the user.

With reference to any one of the second aspect to the second possible implementation manner of the second aspect, in a third possible implementation manner of the second aspect,
the body characteristic value of the user includes one or more of a body temperature value, a heart rate value, a blood glucose value, a blood pressure value, or a blood flow velocity value.

According to the detection method for a wearable device and the wearable device in the embodiments of the present invention, both a value of a distance between a wearable device and a user and a body characteristic value of the user are detected. Only when both the two value of the distance between the wearable device and the user and the body characteristic value of the user have not exceeded a preset threshold range, it can be determined that the user has worn the wearable device. Compared with the prior art, in a manner of determining whether a user wears a wearable device in the embodiments of the present invention, determining accuracy is not affected by a change of an external condition, such as approaching of metal or other objects. Moreover, by means of a human body characteristic value, whether the wearable device is worn on a human body or another object can be precisely distinguished. Therefore, in the embodiments of the present invention, whether the user wears the wearable device can be precisely determined, and a detection success rate is high.

### BRIEF DESCRIPTION OF DRAWINGS

To describe the technical solutions in the embodiments of the present invention or in the prior art more clearly, the following briefly describes the accompanying drawings required for describing the embodiments. Apparently, the accompanying drawings in the following description show merely some embodiments of the present invention, and a person of ordinary skill in the art may still derive other drawings from these accompanying drawings without creative efforts.
FIG. 1 is schematic structural diagram of a smartwatch according to an embodiment of the present invention;
FIG. 2 is schematic structural diagram of smartglasses according to an embodiment of the present invention;
FIG. 3 is schematic structural diagram of a smartband according to an embodiment of the present invention;
FIG. 4 is a schematic flowchart of a detection method for a wearable device according to a first embodiment of the present invention;
FIG. 5 is a schematic flowchart of a detection method for a wearable device according to a second embodiment of the present invention; and
FIG. 6 is schematic structural diagram of a wearable device according to an embodiment of the present invention.

### DESCRIPTION OF EMBODIMENTS

The following clearly and completely describes the technical solutions in the embodiments of the present invention with reference to the accompanying drawings in the embodiments of the present invention. Apparently, the described embodiments are some but not all of the embodiments of the present invention. All other embodiments obtained by a person of ordinary skill in the art based on the embodiments of the present invention without creative efforts shall fall within the protection scope of the present invention.

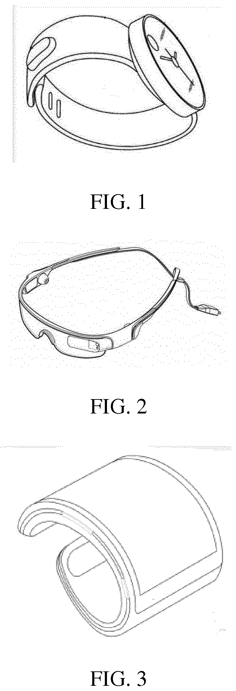

Referring to FIG. 1, FIG. 2, and FIG. 3, during specific implementation, a wearable device described in the embodiments of the present invention may include a smartwatch, a smartband, a smartring, smartglasses, and the like. The foregoing wearable devices are only for an exemplary rather than exhaustive purpose, and the present invention includes but not limited to the foregoing wearable devices.

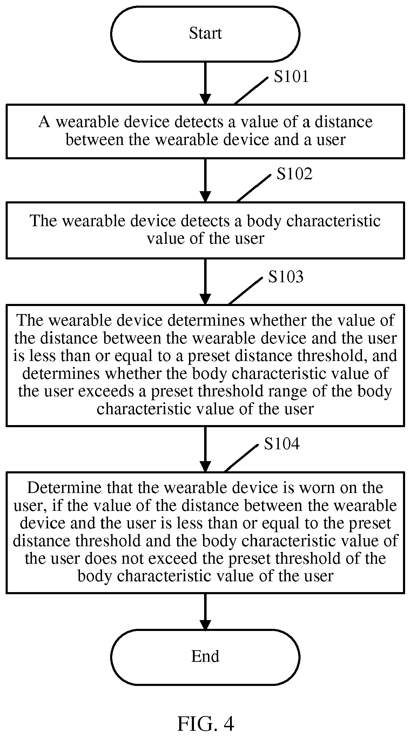

Referring to FIG. 4, FIG. 4 is a schematic flowchart of a detection method for a wearable device according to a first embodiment of the present invention. The method specifically includes the following steps.

S101: A wearable device detects a value of a distance between the wearable device and a user.

Specifically, the wearable device detects the value of the distance between the wearable device and the user by using a distance sensor, and determines, according to the value of the distance between the wearable device and the user, whether the user wears the wearable device. It should be understood that the distance sensor in this embodiment of the present invention include an optical proximity sensor, a linear proximity sensor, an ultrasonic displacement sensor, and the like.

S102: The wearable device detects a body characteristic value of the user.

Specifically, the wearable device obtains the body characteristic value of the user by using a human body characteristic sensor. The body characteristic value of the user includes one or more of a body temperature value, a heart rate value, a blood glucose value, a blood pressure value, or a blood flow velocity value.

For example, the wearable device obtains a body temperature value of the user by using a temperature sensor in the human body characteristic sensor, or obtains a heart rate value of the user by using a heart rate sensor in the human body characteristic sensor, or obtains a blood flow velocity value of the user by using the human body characteristic sensor. Certainly, in another implementation manner of this embodiment of the present invention, body characteristic values such as the blood glucose value and the blood pressure value may be obtained by using the human body characteristic sensor, all of which can reflect a physical status of the user, and these body characteristic values are easy to obtain.

S103: The wearable device determines whether the value of the distance between the wearable device and the user is less than or equal to a preset distance threshold, and determines whether the body characteristic value of the user exceeds a preset threshold range of the body characteristic value of the user.

Specifically, the wearable device obtains the value of the distance between the wearable device and the user, and determines whether the value is less than or equal to the preset distance threshold. It should be understood that a value range of the preset distance threshold is greater than or equal to 0 cm and less than or equal to 2 cm. For example, if the value of the distance between the wearable device and the user obtained by the wearable device is 1.5 cm, the value is less than the preset distance threshold. For another example, if the value of the distance between the wearable device and the user obtained by the wearable device is 2.5 cm, the value is greater than the preset distance threshold.

It needs to be known that the preset threshold of the body characteristic value of the user includes preset thresholds of the body temperature value, the heart rate value, the blood glucose value, the blood pressure value, the blood flow velocity value, and the like. For example, a value range of the preset threshold of the body temperature value of the user is 36°C to 38°C. If the body temperature value of the user obtained by using the temperature sensor is 35°C, the body characteristic value of the user exceeds the value range of the preset threshold of the body characteristic value of the user. For another example, a value range of the preset threshold of the heart rate value is 60 times to 100 times per minute. If the heart rate value of the user obtained by the heart rate sensor in the human body characteristic sensor is 75 times per minute, the body characteristic value of the user does not exceed the value range of the preset threshold of the body characteristic value of the user. For still another example, a value range of a preset threshold of a forearm blood flow velocity value of the user is 6 ml/min/100g to 10 ml/min/100g. When the forearm skin blood flow velocity value of the user detected by the wearable device is 8 ml/min/100g, the body characteristic value of the user does not exceed the preset threshold range of the body characteristic value of the user. Similarly, thresholds of the blood pressure value and the blood glucose value may be preset. After obtaining the foregoing body characteristic values, the wearable device compares the body characteristic values with the preset thresholds, to determine whether the body characteristic values exceed the preset threshold ranges. According to an actual requirement, one or more of the body characteristic values of the user may be obtained.

S104: Determine that the wearable device is worn on the user, if the value of the distance between the wearable device and the user is less than or equal to the preset distance threshold and the body characteristic value of the user does not exceed the preset threshold of the body characteristic value of the user.

Specifically, the wearable device obtains the value of the distance between the wearable device and the user, the value range of the preset distance threshold is greater than or equal to 0 cm and less than or equal to 2 cm, and the value range of the preset threshold of the body temperature value is 36°C to 38°C. If the value of the distance between the wearable device and the user obtained by the wearable device is 1.5 cm, the value is less than the preset distance threshold. If the body temperature value of the user obtained by the wearable device by using the temperature sensor is 37.3°C, the body characteristic value of the user does not exceed the preset threshold range of the body characteristic value of the user. According to the value of the distance between the wearable device and the user and the body temperature value of the user, it is determined that the wearable device is worn on the user.

It needs to be known that only when the value of the distance between the wearable device and the user is less than or equal to the preset distance threshold, and the body characteristic value of the user does not exceed the preset threshold of the body characteristic value of the user, it can be determined that the wearable device is worn on the user. If the value of the distance between the wearable device and the user is less than or equal to the preset distance threshold, but the body characteristic value of the user exceeds the preset threshold of the body characteristic value of the user, it cannot be determined that the wearable device is worn on the user. Similarly, if the value of the distance between the wearable device and the user is greater than the preset distance threshold, but the body characteristic value of the user does not exceed the preset threshold of the body characteristic value of the user, it cannot be determined that the wearable device is worn on the user, either.

According to the detection method for a wearable device and the wearable device in this embodiment of the present invention, both a value of a distance between a wearable device and a user and a body characteristic value of the user are detected. Only when both the two value of the distance between the wearable device and the user and the body characteristic value of the user have not exceeded a preset threshold range, it can be determined that the user has worn the wearable device. Compared with the prior art, in a manner of determining whether a user wears a wearable device in this embodiment of the present invention, determining accuracy is not affected by a change of an external condition, such as approaching of metal or other objects. Moreover, by means of a human body characteristic value, whether the wearable device is worn on a human body or another object can be precisely distinguished. Therefore, in this embodiment of the present invention, whether the user wears the wearable device can be precisely determined, and a detection success rate is high.

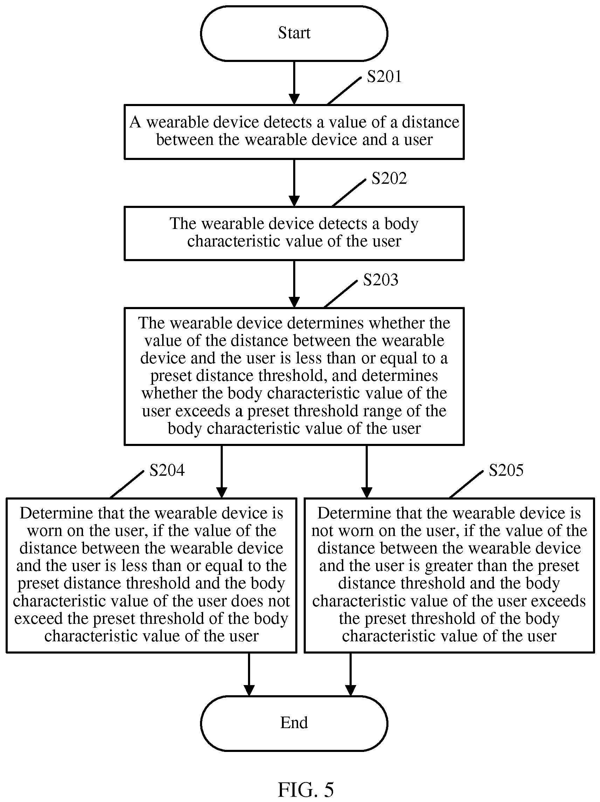

Referring to FIG. 5, FIG. 5 is a schematic flowchart of a detection method for a wearable device according to a second embodiment of the present invention. The method specifically includes the following steps.

S201: A wearable device detects a value of a distance between the wearable device and a user.

Specifically, the wearable device detects the value of the distance between the wearable device and the user by using a distance sensor or an optical proximity sensor, and determines, according to the value of the distance between the wearable device and the user, whether the user wears the wearable device. It should be understood that the distance sensor in this embodiment of the present invention includes the optical proximity sensor, a linear proximity sensor, an ultrasonic displacement sensor, and the like.

S202: The wearable device detects a body characteristic value of the user.

Specifically, the wearable device obtains the body characteristic value of the user by using a human body characteristic sensor. The body characteristic value of the user includes one or more of a body temperature value, a heart rate value, a blood glucose value, a blood pressure value, or a blood flow velocity value. For example, the wearable device obtains a body temperature value of the user by using a temperature sensor in the human body characteristic sensor, or obtains a heart rate value of the user by using a heart rate sensor in the human body characteristic sensor, or obtains a blood flow velocity value of the user by using the human body characteristic sensor. Certainly, in another implementation manner of this embodiment of the present invention, body characteristic values such as the blood glucose value and the blood pressure value may be obtained by using the human body characteristic sensor, all of which can reflect a physical status of the user, and these body characteristic values are easy to obtain.

S203: The wearable device determines whether the value of the distance between the wearable device and the user is less than or equal to a preset distance threshold, and determines whether the body characteristic value of the user exceeds a preset threshold range of the body characteristic value of the user.

Specifically, the wearable device obtains the value of the distance between the wearable device and the user, and determines whether the value is less than or equal to the preset distance threshold. It should be understood that a value range of the preset distance threshold is greater than or equal to 0 cm and less than or equal to 2 cm. For example, if the value of the distance between the wearable device and the user obtained by the wearable device is 1.5 cm, the value is less than the preset distance threshold. For another example, if the value of the distance between the wearable device and the user obtained by the wearable device is 2.5 cm, the value is greater than the preset distance threshold.

It needs to be known that the preset threshold of the body characteristic value of the user includes preset thresholds of the body temperature value, the heart rate value, the blood glucose value, the blood pressure value, the blood flow velocity value, and the like. For example, a value range of the preset threshold of the body temperature value of the user is 36°C to 38°C. If the body temperature value of the user obtained by using the temperature sensor is 35°C, the body characteristic value of the user exceeds the value range of the preset threshold of the body characteristic value of the user. For another example, a value range of the preset threshold of the heart rate value is 60 times to 100 times per minute. If the heart rate value of the user obtained by the heart rate sensor in the human body characteristic sensor is 75 times per minute, the body characteristic value of the user does not exceed the value range of the preset threshold of the body characteristic value of the user. For still another example, a value range of a preset threshold of a forearm blood flow velocity value of the user is 6 ml/min/100g to 10 ml/min/100g. When the forearm skin blood flow velocity value of the user detected by the wearable device is 8 ml/min/100g, the body characteristic value of the user does not exceed the preset threshold range of the body characteristic value of the user. Similarly, thresholds of the blood pressure value and the blood glucose value may be preset. After obtaining the foregoing body characteristic values, the wearable device compares the body characteristic values with the preset thresholds, to determine whether the body characteristic values exceed the preset threshold ranges. According to an actual requirement, one or more of the body characteristic values of the user may be obtained.

S204: Determine that the wearable device is worn on the user, if the value of the distance between the wearable device and the user is less than or equal to the preset distance threshold and the body characteristic value of the user does not exceed the preset threshold of the body characteristic value of the user.

Specifically, the wearable device obtains the value of the distance between the wearable device and the user, the value range of the preset distance threshold is greater than or equal to 0 cm and less than or equal to 2 cm, and the value range of the preset threshold of the body temperature value is 36°C to 38°C. If the value of the distance between the wearable device and the user obtained by the wearable device is 1.5 cm, the value is less than the preset distance threshold. If the body temperature value of the user obtained by the wearable device by using the temperature sensor is 37.3°C, the body characteristic value of the user does not exceed the preset threshold range of the body characteristic value of the user. According to the value of the distance between the wearable device and the user and the body temperature value of the user, it is determined that the wearable device is worn on the user.

S205: Determine that the wearable device is not worn on the user, if the value of the distance between the wearable device and the user is greater than the preset distance threshold, and if the body characteristic value of the user exceeds the preset threshold of the body characteristic value of the user.

Specifically, only when the value of the distance between the wearable device and the user is less than or equal to the preset distance threshold, and the body characteristic value of the user does not exceed the preset threshold of the body characteristic value of the user, it can be determined that the wearable device is worn on the user. If the value of the distance between the wearable device and the user is less than or equal to the preset distance threshold, but the body characteristic value of the user exceeds the preset threshold of the body characteristic value of the user, it cannot be determined that the wearable device is worn on the user. Similarly, if the value of the distance between the wearable device and the user is greater than the preset distance threshold, but the body characteristic value of the user does not exceed the preset threshold of the body characteristic value of the user, it cannot be determined that the wearable device is worn on the user, either.

For example, the value range of the preset distance threshold is greater than or equal to 0 cm and less than or equal to 2 cm, and the value range of the preset threshold of the body temperature value is 36°C to 38°C. If the value of the distance between the wearable device and the user obtained by the wearable device is 2.3 cm, the value is greater than the preset distance threshold. If the body temperature value of the user obtained by the wearable device by using the temperature sensor is 37°C, the body characteristic value of the user does not exceed the preset threshold range of the body characteristic value of the user. Therefore, it is determined that the wearable device is not worn on the user. For another example, the value range of the preset distance threshold is greater than or equal to 0 cm and less than or equal to 2 cm, and the value range of the preset threshold of the heart rate value is 60 times to 100 times per minute. If the value of the distance between the wearable device and the user obtained by the wearable device is 2 cm, the value is equal to the preset distance threshold, and if the heart rate value of the user obtained by the heart rate sensor in the human body characteristic sensor is 50 times per minute, it is determined that the wearable device is not worn on the user.

According to the detection method for a wearable device and the wearable device in this embodiment of the present invention, both a value of a distance between a wearable device and a user and a body characteristic value of the user are detected. Only when both the two value of the distance between the wearable device and the user and the body characteristic value of the user have not exceeded a preset threshold range, it can be determined that the user has worn the wearable device. Compared with the prior art, in a manner of determining whether a user wears a wearable device in this embodiment of the present invention, determining accuracy is not affected by a change of an external condition, such as approaching of metal or other objects. Moreover, by means of a human body characteristic value, whether the wearable device is worn on a human body or another object can be precisely distinguished. Therefore, in this embodiment of the present invention, whether the user wears the wearable device can be precisely determined, and a detection success rate is high.

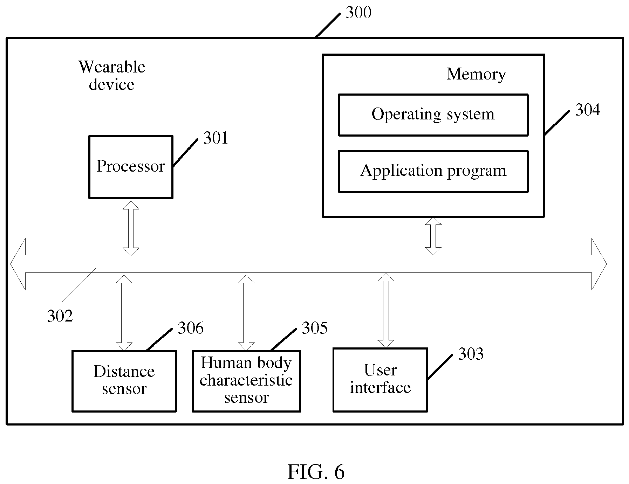

Refer to FIG. 6, which is schematic structural diagram of a wearable device according to an embodiment of the present invention. A wearable device 300 includes at least one processor 301, at least one human body characteristic sensor 305, and at least one distance sensor 306, and further includes at least one user interface 303, at least one communications bus 302, and at least one memory 304.

The processor 301 may include a CPU. The human body characteristic sensor 305 may include a body temperature sensor, a heart rate sensor, a blood glucose sensor, a blood pressure sensor, a blood flow velocity sensor, and the like. The distance sensor 306 may include an optical proximity sensor, a linear proximity sensor, an ultrasonic displacement sensor, and the like. The communications bus 302 is configured to implement connection and communication between these components. The user interface 303 may include a display (Display), a keyboard (Keyboard), and optionally, the user interface 303 may further include a standard wired interface and wireless interface. The memory 304 may be a high-speed RAM memory, or may be a non-volatile memory (non-volatile memory), such as at least one magnetic disk memory. Optionally, the memory 304 may further include at least one storage apparatus that is located far away from the processor 301. The memory 304 stores a set of program code, and the processor 301 is configured to invoke the program code stored in the memory 304 to execute the following operations:

The memory 304 stores a set of program code, and the processor 301 is configured to invoke the program code stored in the memory 304 to execute the following operations:
the distance sensor is configured to detect a value of a distance between the wearable device and a user;
the human body characteristic sensor is configured to detect a body characteristic value of the user; and
the processor is configured to: determine whether the value of the distance between the wearable device and the user is less than or equal to a preset distance threshold; determine whether the body characteristic value of the user exceeds a preset threshold range of the body characteristic value of the user; and determine that the wearable device is worn on the user, if the value of the distance between the wearable device and the user is less than or equal to the preset distance threshold and the body characteristic value of the user does not exceed the preset threshold of the body characteristic value of the user.

Specifically, the wearable device detects the value of the distance between the wearable device and the user by using the distance sensor or the optical proximity sensor, and determines, according to the value of the distance between the wearable device and the user, whether the user wears the wearable device.

The wearable device obtains the body characteristic value of the user by using the human body characteristic sensor. The body characteristic value of the user includes one or more of a body temperature value, a heart rate value, a blood glucose value, a blood pressure value, or a blood flow velocity value. For example, the wearable device obtains a body temperature value of the user by using a temperature sensor in the human body characteristic sensor, or obtains a heart rate value of the user by using a heart rate sensor in the human body characteristic sensor, or obtains a blood flow velocity value of the user by using the human body characteristic sensor. Certainly, in another implementation manner of this embodiment of the present invention, body characteristic values such as the blood glucose value and the blood pressure value may be obtained by using the human body characteristic sensor, all of which can reflect a physical status of the user, and these body characteristic values are easy to obtain.

In an optional embodiment, the processor 301 may invoke the program code stored in the memory 304 to further execute the following operations:
determining that the wearable device is not worn on the user, if the value of the distance between the wearable device and the user is greater than the preset distance threshold.

In an optional embodiment, the processor 301 may invoke the program code stored in the memory 304 to further execute the following operations:
determining that the wearable device is not worn on the user, if the body characteristic value of the user exceeds the preset threshold of the body characteristic value of the user.

In an optional embodiment, the processor 301 may invoke the program code stored in the memory 304 to further execute the following operations:
the body characteristic value of the user includes one or more of a body temperature value, a heart rate value, a blood glucose value, a blood pressure value, or a blood flow velocity value.

Specifically, the wearable device obtains the value of the distance between the wearable device and the user, and determines whether the value is less than or equal to the preset distance threshold. It should be understood that a value range of the preset distance threshold is greater than or equal to 0 cm and less than or equal to 2 cm. For example, if the value of the distance between the wearable device and the user obtained by the wearable device is 1.5 cm, the value is less than the preset distance threshold. For another example, if the value of the distance between the wearable device and the user obtained by the wearable device is 2.5 cm, the value is greater than the preset distance threshold.

It needs to be known that the preset threshold of the body characteristic value of the user includes preset thresholds of the body temperature value, the heart rate value, the blood glucose value, the blood pressure value, the blood flow velocity value, and the like. For example, a value range of the preset threshold of the body temperature value of the user is 36°C to 38°C. If the body temperature value of the user obtained by using the temperature sensor is 35°C, the body characteristic value of the user exceeds the value range of the preset threshold of the body characteristic value of the user. For another example, a value range of the preset threshold of the heart rate value is 60 times to 100 times per minute. If the heart rate value of the user obtained by the heart rate sensor in the human body characteristic sensor is 75 times per minute, the body characteristic value of the user does not exceed the value range of the preset threshold of the body characteristic value of the user. For still another example, a value range of a preset threshold of a forearm blood flow velocity value of the user is 6 ml/min/100g to 10 ml/min/100g. When the forearm skin blood flow velocity value of the user detected by the wearable device is 8 ml/min/100g, the body characteristic value of the user does not exceed the preset threshold range of the body characteristic value of the user. Similarly, thresholds of the blood pressure value and the blood glucose value may be preset. After obtaining the foregoing body characteristic values, the wearable device compares the body characteristic values with the preset thresholds, to determine whether the body characteristic values exceed the preset threshold ranges. According to an actual requirement, one or more of the body characteristic values of the user may be obtained.

In an optional embodiment, the processor 301 may invoke the program code stored in the memory 304 to further execute the following operations:
the value of the distance between the wearable device and the user is obtained by using the distance sensor.

In an optional embodiment, the processor 301 may invoke the program code stored in the memory 304 to further execute the following operations:
the body characteristic value of the user is obtained by using the human body characteristic sensor.

Specifically, the wearable device obtains the value of the distance between the wearable device and the user, the value range of the preset distance threshold is greater than or equal to 0 cm and less than or equal to 2 cm, and the value range of the preset threshold of the body temperature value is 36°C to 38°C. If the value of the distance between the wearable device and the user obtained by the wearable device is 1.5 cm, the value is less than the preset distance threshold. If the body temperature value of the user obtained by the wearable device by using the temperature sensor is 37.3°C, the body characteristic value of the user does not exceed the preset threshold range of the body characteristic value of the user. According to the value of the distance between the wearable device and the user and the body temperature value of the user, it is determined that the wearable device is worn on the user.

Only when the value of the distance between the wearable device and the user is less than or equal to the preset distance threshold, and the body characteristic value of the user does not exceed the preset threshold of the body characteristic value of the user, it can be determined that the wearable device is worn on the user. If the value of the distance between the wearable device and the user is less than or equal to the preset distance threshold, but the body characteristic value of the user exceeds the preset threshold of the body characteristic value of the user, it cannot be determined that the wearable device is worn on the user. Similarly, if the value of the distance between the wearable device and the user is greater than the preset distance threshold, but the body characteristic value of the user does not exceed the preset threshold of the body characteristic value of the user, it cannot be determined that the wearable device is worn on the user, either.

For example, the value range of the preset distance threshold is greater than or equal to 0 cm and less than or equal to 2 cm, and the value range of the preset threshold of the body temperature value is 36°C to 38°C. If the value of the distance between the wearable device and the user obtained by the wearable device is 2.3 cm, the value is greater than the preset distance threshold. If the body temperature value of the user obtained by the wearable device by using the temperature sensor is 37°C, the body characteristic value of the user does not exceed the preset threshold range of the body characteristic value of the user. Therefore, it is determined that the wearable device is not worn on the user. For another example, the value range of the preset distance threshold is greater than or equal to 0 cm and less than or equal to 2 cm, and the value range of the preset threshold of the heart rate value is 60 times to 100 times per minute. If the value of the distance between the wearable device and the user obtained by the wearable device is 2 cm, the value is equal to the preset distance threshold, and if the heart rate value of the user obtained by the heart rate sensor in the human body characteristic sensor is 50 times per minute, it is determined that the wearable device is not worn on the user.

According to the detection method for a wearable device and the wearable device in the embodiments of the present invention, both a value of a distance between a wearable device and a user and a body characteristic value of the user are detected. Only when both the two value of the distance between the wearable device and the user and the body characteristic value of the user have not exceeded a preset threshold range, it can be determined that the user has worn the wearable device. Compared with the prior art, in a manner of determining whether a user wears a wearable device in this embodiment of the present invention, determining accuracy is not affected by a change of an external condition, such as approaching of metal or other objects. Moreover, by means of a human body characteristic value, whether the wearable device is worn on a human body or another object can be precisely distinguished. Therefore, in this embodiment of the present invention, whether the user wears the wearable device can be precisely determined, and a detection success rate is high.

A person of ordinary skill in the art may understand that, each aspect of the present invention or a possible implementation manner of each aspect may be specifically implemented as a system, a method, or a computer program product. In addition, each aspect of the present invention or the possible implementation manner of each aspect may take a form of a computer program product, and the computer program product refers to computer-readable program code stored in a computer-readable medium.

The computer-readable medium may be a computer-readable data medium or a computer-readable storage medium. The computer-readable storage medium includes but is not limited to an electronic, magnetic, optical, electromagnetic, infrared, or semi-conductive system, device, or apparatus, or any appropriate combination thereof, such as a random access memory (RAM), a read-only memory (ROM), an erasable programmable read only memory (EPROM or flash memory), an optical fiber, and a compact disc read only memory (CD-ROM).

A processor in a computer reads computer-readable program code stored in a computer-readable medium, so that the processor can perform a function and an action specified in each step or a combination of steps in a flowchart; an apparatus is generated to implement a function and an action specified in each block or a combination of blocks in a block diagram.

All computer-readable program code may be executed on a user computer, or some may be executed on a user computer as a standalone software package, or some may be executed on a local computer of a user while some is executed on a remote computer, or all the code may be executed on a remote computer or a server. It should also be noted that, in some alternative implementation solutions, each step in the flowcharts or functions specified in each block in the block diagrams may not occur in the illustrated order. For example, two consecutive steps or two blocks in the illustration, which are dependent on an involved function, may in fact be executed substantially at the same time, or these blocks may sometimes be executed in reverse order.

Obviously, a person skilled in the art can make various modifications and variations to the present invention without departing from the scope of the present invention. The present invention is intended to cover these modifications and variations provided that they fall within the scope of protection defined by the following claims.

## Claims

1. A detection method for a wearable device (300), said wearable device comprising a distance sensor (306), a human body characteristic sensor (305) and a processor (301), the method comprising:
detecting, by said distance sensor (306), a value of a distance between the wearable device and a user;
detecting, by said human body characteristic sensor (305), a body characteristic value of the user;
determining, by said processor (301), whether the value of the distance between the wearable device and the user is less than or equal to a preset distance threshold; and
determining, by the processor (301), whether the body characteristic value of the user exceeds a preset threshold range of the body characteristic value of the user;
the method further comprising determining, by the processor (301), that
the wearable device is worn on the user, if the value of the distance between the wearable device and the user is less than or equal to the preset distance threshold and the body characteristic value of the user does not exceed the preset threshold of the body characteristic value of the user.

2. The method according to claim 1, wherein the method further comprises:
determining, by the processor (301), that the wearable device is not worn on the user, if the value of the distance between the wearable device and the user is greater than the preset distance threshold.

3. The method according to claim 1, wherein the method further comprises:
determining, by the processor (301), that the wearable device is not worn on the user, if the body characteristic value of the user exceeds the preset threshold of the body characteristic value of the user.

4. The method according to any one of claims 1 to 3, wherein
the body characteristic value of the user comprises one or more of a body temperature value, a heart rate value, a blood glucose value, a blood pressure value, or a blood flow velocity value.

5. A wearable device (300), comprising: a distance sensor (306), a human body characteristic sensor (305), and a processor (301), wherein
the distance sensor (306) is configured to detect a value of a distance between the wearable device and a user; and
the human body characteristic sensor (305) is configured to detect a body characteristic value of the user; wherein the processor (301) is configured to: determine whether the value of the distance between the wearable device and the user is less than or equal to a preset distance threshold; determine whether the body characteristic value of the user exceeds a preset threshold range of the body characteristic value of the user; and determine that the wearable device is worn on the user, if the value of the distance between the wearable device and the user is less than or equal to the preset distance threshold and the body characteristic value of the user does not exceed the preset threshold of the body characteristic value of the user.

6. The wearable device according to claim 5, wherein the processor (301) is further configured to determine that the wearable device is not worn on the user, if the value of the distance between the wearable device and the user exceeds the preset distance threshold.

7. The wearable device according to claim 5, wherein the processor (301) is further configured to determine that the wearable device is not worn on the user, if the body characteristic value of the user exceeds the preset threshold of the body characteristic value of the user.

8. The wearable device according to any one of claims 5 to 7, wherein
the body characteristic value of the user comprises one or more of a body temperature value, a heart rate value, a blood glucose value, a blood pressure value, or a blood flow velocity value.

## Patentansprüche

1. Erfassungsverfahren für ein tragbares Gerät (300), wobei das tragbare Gerät einen Abstandssensor (306), einen Menschenkörper-Eigenschaftssensor (305) und einen Prozessor (301) umfasst und das Verfahren Folgendes umfasst:
Erfassen, durch den Abstandssensor (306), eines Werts eines Abstands zwischen dem tragbaren Gerät und einem Benutzer;
Erfassen, durch den Menschenkörper-Eigenschaftssensor (305), eines Körpereigenschaftswerts des Benutzers;
Bestimmen, durch den Prozessor (301), ob der Wert des Abstands zwischen dem tragbaren Gerät und dem Benutzer kleiner als oder gleich einem voreingestellten Abstandsschwellenwert ist; und
Bestimmen, durch den Prozessor (301), ob der Körpereigenschaftswert des Benutzers einen voreingestellten Schwellenwertbereich des Körpereigenschaftswerts des Benutzers überschreitet;
das Verfahren ferner Folgendes umfasst: Bestimmen, durch den Prozessor (301), dass das tragbare Gerät vom Benutzer getragen wird, falls der Wert des Abstands zwischen dem tragbaren Gerät und dem Benutzer kleiner als oder gleich dem voreingestellten Abstandsschwellenwert ist und der Körpereigenschaftswert des Benutzers nicht den voreingestellten Schwellenwert des Körpereigenschaftswerts des Benutzer überschreitet.

2. Verfahren gemäß Anspruch 1, wobei das Verfahren ferner Folgendes umfasst:
Bestimmen, durch den Prozessor (301), dass das tragbare Gerät nicht vom Benutzer getragen wird, falls der Wert des Abstands zwischen dem tragbaren Gerät und dem Benutzer größer als der voreingestellte Abstandsschwellenwert ist.

3. Verfahren gemäß Anspruch 1, wobei das Verfahren ferner Folgendes umfasst:
Bestimmen, durch den Prozessor (301), dass das tragbare Gerät nicht vom Benutzer getragen wird, falls der Körpereigenschaftswert des Benutzers den voreingestellten Schwellenwert des Körpereigenschaftswerts des Benutzers überschreitet.

4. Verfahren gemäß einem der Ansprüche 1 bis 3, wobei der Körpereigenschaftswert des Benutzers einen oder mehrere eines Körpertemperaturwerts, eines Herzfrequenzwerts, eines Blutzuckerwerts, eines Blutdruckwerts oder eines Blutströmungsgeschwindigkeitswerts umfasst.

5. Tragbares Gerät (300), das Folgendes umfasst: einen Abstandssensor (306), einen Menschenkörper-Eigenschaftssensor (305) und einen Prozessor (301), wobei der Abstandssensor (306) dazu ausgelegt ist, einen Wert eines Abstands zwischen dem tragbaren Gerät und einem Benutzer zu erfassen; und
der Menschenkörper-Eigenschaftssensor (305) dazu ausgelegt ist, einen Körpereigenschaftswert des Benutzers zu erfassen; wobei
der Prozessor (301) für Folgendes ausgelegt ist: Bestimmen, ob der Wert des Abstands zwischen dem tragbaren Gerät und dem Benutzer kleiner als oder gleich einem voreingestellten Abstandsschwellenwert ist; Bestimmen, ob der Körpereigenschaftswert des Benutzers einen voreingestellten Schwellenwertbereich des Körpereigenschaftswerts des Benutzers überschreitet; und Bestimmen, dass das tragbare Gerät vom Benutzer getragen wird, falls der Wert des Abstands zwischen dem tragbaren Gerät und dem Benutzer kleiner als oder gleich dem voreingestellten Abstandsschwellenwert ist und der Körpereigenschaftswert des Benutzers nicht den voreingestellten Schwellenwert des Körpereigenschaftswerts des Benutzers überschreitet.

6. Tragbares Gerät gemäß Anspruch 5, wobei der Prozessor (301) ferner dazu ausgelegt ist zu bestimmen, dass das tragbare Gerät nicht vom Benutzer getragen wird, falls der Wert des Abstands zwischen dem tragbaren Gerät und dem Benutzer den voreingestellten Abstandsschwellenwert überschreitet.

7. Tragbares Gerät gemäß Anspruch 5, wobei der Prozessor (301) ferner dazu ausgelegt ist zu bestimmen, dass das tragbare Gerät nicht vom Benutzer getragen wird, falls der Körpereigenschaftswert des Benutzers den voreingestellten Schwellenwert des Körpereigenschaftswerts des Benutzers überschreitet.

8. Tragbares Gerät gemäß einem der Ansprüche 5 bis 7, wobei der Körpereigenschaftswert des Benutzers einen oder mehrere eines Körpertemperaturwerts, eines Herzfrequenzwerts, eines Blutzuckerwerts, eines Blutdruckwerts oder eines Blutströmungsgeschwindigkeitswerts umfasst.

## Revendications

1. Procédé de détection pour un dispositif pouvant être porté sur soi (300), ledit dispositif pouvant être porté sur soi comprenant un capteur de distance (306), un capteur de caractéristiques du corps humain (305) et un processeur (301), le procédé comprenant les étapes consistant à :
détecter, par ledit capteur de distance (306), une valeur de distance entre le dispositif pouvant être porté sur soi et un utilisateur ;
détecter, par ledit capteur de caractéristique du corps humain (305), une valeur caractéristique du corps de l'utilisateur ;
déterminer, par ledit processeur (301), si la valeur de la distance entre le dispositif pouvant être porté sur soi et l'utilisateur est inférieure ou égale à un seuil de distance prédéfini ; et déterminer, par le processeur (301), si la valeur caractéristique du corps de l'utilisateur dépasse une plage de seuil prédéfinie de la valeur caractéristique du corps de l'utilisateur ; le procédé comprenant en outre la détermination, par le processeur (301), du fait que le dispositif pouvant être porté sur soi est porté par l'utilisateur, si la valeur de la distance entre le dispositif pouvant être porté sur soi et l'utilisateur est inférieure ou égale au seuil de distance prédéfini et la valeur caractéristique du corps de l'utilisateur ne dépasse pas le seuil prédéfini de la valeur caractéristique du corps de l'utilisateur.

2. Procédé selon la revendication 1, dans lequel le procédé comprend en outre l'étape consistant à:
déterminer, par le processeur (301), que le dispositif pouvant être porté sur soi n'est pas porté par l'utilisateur, si la valeur de la distance entre le dispositif pouvant être porté sur soi et l'utilisateur est supérieure au seuil de distance prédéfini.

3. Procédé selon la revendication 1, dans lequel le procédé comprend en outre l'étape consistant à:
déterminer, par le processeur (301), que le dispositif pouvant être porté sur soi n'est pas porté sur l'utilisateur, si la valeur caractéristique du corps de l'utilisateur dépasse le seuil prédéfini de la valeur caractéristique du corps de l'utilisateur.

4. Procédé selon l'une quelconque des revendications 1 à 3, dans lequel :
la valeur caractéristique du corps de l'utilisateur comprend une ou plusieurs valeurs parmi une valeur de température corporelle, une valeur de fréquence cardiaque, une valeur de glycémie, une valeur de pression sanguine ou une valeur de vitesse d'écoulement sanguin.

5. Dispositif pouvant être porté sur soi (300), comprenant : un capteur de distance (306), un capteur de caractéristiques du corps humain (305) et un processeur (301), dans lequel
le capteur de distance (306) est configuré pour détecter une valeur de distance entre le dispositif pouvant être porté sur soi et un utilisateur ; et le capteur de caractéristiques du corps humain (305) est configuré pour détecter une valeur caractéristique du corps de l'utilisateur ;
dans lequel le processeur (301) est configuré pour : déterminer si la valeur de la distance entre le dispositif pouvant être porté sur soi et l'utilisateur est inférieure ou égale à un seuil de distance prédéfini ; déterminer si la valeur caractéristique du corps de l'utilisateur dépasse une plage de seuil prédéfinie de la valeur caractéristique du corps de l'utilisateur ; et déterminer que le dispositif pouvant être porté sur soi est porté par l'utilisateur, si la valeur de la distance entre le dispositif pouvant être porté sur soi et l'utilisateur est inférieure ou égale au seuil de distance prédéfini et la valeur caractéristique du corps de l'utilisateur ne dépasse pas le seuil prédéfini de la valeur caractéristique du corps de l'utilisateur.

6. Dispositif pouvant être porté sur soi selon la revendication 5, dans lequel le processeur (301) est en outre configuré pour déterminer que le dispositif pouvant être porté sur soi n'est pas porté par l'utilisateur, si la valeur de la distance entre le dispositif pouvant être porté sur soi et l'utilisateur dépasse le seuil de distance prédéfini.

7. Dispositif pouvant être porté sur soi selon la revendication 5, dans lequel le processeur (301) est en outre configuré pour déterminer que le dispositif pouvant être porté sur soi n'est pas porté par l'utilisateur, si la valeur caractéristique du corps de l'utilisateur dépasse le seuil prédéfini de la valeur caractéristique du corps de l'utilisateur.

8. Dispositif pouvant être porté sur soi selon l'une quelconque des revendications 5 à 7, dans lequel
la valeur caractéristique du corps de l'utilisateur comprend une ou plusieurs valeurs parmi une valeur de température corporelle, une valeur de fréquence cardiaque, une valeur de glycémie, une valeur de pression sanguine ou une valeur de vitesse d'écoulement sanguin.
